# EUROPÄISCHE PATENTSCHRIFT

(11) **EP 0 944 941 B1**
(45) Veröffentlichungstag und Bekanntmachung des Hinweises auf die Patenterteilung: **04.09.2002**
(21) Anmeldenummer: 97952915.3
(22) Anmeldetag: 11.12.1997
(51) Int. Cl.: H01T 23/00, B60H 3/00

(54) **VORRICHTUNG ZUR ERZEUGUNG AKTIVER SAUERSTOFFIONEN IN DER LUFT FÜR DIE LUFTVERBESSERUNG, MIT EINEM LUFTIONISATOR**
DEVICE TO PRODUCE ACTIVE OXYGEN IONS IN THE AIR FOR IMPROVED AIR QUALITY
DISPOSITIF DE PRODUCTION D'IONS D'OXYGENE ACTIFS DANS L'AIR POUR LA PURIFICATION DE L'AIR, AU MOYEN D'UN IONISATEUR D'AIR

(30) Priorität: 11.12.1996 DE 19651402
(43) Veröffentlichungstag der Anmeldung: 29.09.1999
(73) Patentinhaber: T.E.M.! Technische Entwicklungen und Management GmbH, 63840 Hausen (DE)
(72) Erfinder: RUMP, Hanns, D-63840 Hausen (DE); KIESEWETTER, Olaf, D-98716 Geschwenda (DE)
(74) Vertreter: Mierswa, Klaus, Dipl.-Ing.
(86) Internationale Anmeldenummer: EP9706925
(87) Internationale Veröffentlichungsnummer: WO98026482

(56) Entgegenhaltungen:
- WO-A-93/22603
- CH-A- 600 250
- GB-A- 2 304 576
- US-A- 4 901 194
- US-A- 4 918 568
- PATENT ABSTRACTS OF JAPAN vol. 010, no. 029 (M-451), 5.Februar 1986 & JP 60 185623 A (MATSUSHITA DENKI SANGYO KK), 21.September 1985,

## Beschreibung

Die Erfindung betrifft eine Vorrichtung zur Erzeugung aktiver Sauerstoffionen in der Luft für die Luftverbesserung, insbesondere von Atemluft, bestehend aus wenigstens einem Luftionisator und einem eine zur Luftionisierung ausreichende elektrische Hochspannung erzeugenden elektrischen Transformator.

Gesunde Atemluft wird beschrieben als Luft ohne wesentliche Anteile schädlicher Gase oder bemerkbarer, vielfach unangenehmer Geruchsstoffe. Gesunde Atemluft soll eine möglichst geringe Anzahl von Bakterien, Viren und anderen Keimen enthalten, was sehr wichtig ist, besonders in Kenntnis der Tatsache, daß durch Luft übertragene Infektionen, z.B. in Krankenhäusern, Hotels, Restaurants und Massenverkehrsmitteln, in Deutschland in jedem Jahr mehr als 40.000 Menschen mit Todesfolge erkranken, wie eine wissenschaftliche Studie des Robert-Koch-Institutes feststellte (Bundesgesundheitsblatt Heft 7/96, Seite 246). Die Kosten dieser nosokominalen Infektionen werden von den Autoren auf mehr als 3 Mrd. DM jährlich geschätzt.

Es ist eine bekannte Tatsache, daß Luft mit hohen Anteilen an Geruchsstoffen, den Komfort, die Kondition und Konzentrationsfähigkeit und somit die Lebensqualität der exponierten Menschen drastisch mindert. Es ist leicht einsehbar, daß z.B. der Gestank von Kerosin oder anderen Motorabgasen in Restaurants z.B. an Flughäfen oder in Straßennähe, den Genuß feiner Speisen unmöglich macht oder jedenfalls erheblich mindert, weil die Geschmacks- und Geruchsnerven durch die Grundbelastung an Gestank derart blockiert sind, daß sie keine Nuancen mehr aufnehmen können. Es ist auch bekannt, daß der dauernde Aufenthalt in hochbelasteter Luft müde und abgespannt macht. Menschen, die in schlechter Luft arbeiten müssen, machen nach einiger Zeit signifikant mehr Fehler als Menschen, die in einwandfreier Luft arbeiten. Ebenso ist bekannt, daß elektrostatische Aufladungen in vermehrtem Umfang entstehen, wenn die im Raum befindliche Luft arm ist an Ionen bzw. wenn positiv oder negativ geladene Ionen dominieren. Solche Luft, volkstümlich als "elektrisch geladen" bezeichnet, hat unbestritten Einfluß auf das vegetative Nervensystem. Des Weiteren kann es durch statische Aufladungen zu Beschädigungen an elektronischen Geräten und Datenträgern kommen. Ebenso ist der Krankenstand in Unternehmen, die ihren Mitarbeitern durch schlecht funktionierende Klimaanlagen keine gute Atemluft anbieten können, deutlich höher als der von Unternehmen, in denen auf einwandfreie Luft Wert gelegt wird.

In Kraftfahrzeugen werden die Passagiere durch die Abgase anderer Fahrzeuge belästigt und mitunter gesundheitlich geschädigt. In der Zeitschrift "Scientist", Ausgabe September 1996, wird in diesem Zusammenhang eine dänische Untersuchung zitiert, der zufolge das Risiko von Busfahrern, an Lungenkrebs zu erkranken, um 50 % höher ist als in einer Vergleichsgruppe. Sinnvolle bekannte Maßnahmen zur Verringerung der Immission der Fahrzeugpassagiere sind z.B. sensorgesteuerte Umluftsysteme, bei denen das Einströmen von Außenluft stets dann gestoppt und auf Umluftbetrieb umgeschaltet wird, wenn das Fahrzeug eine Zone erhöhter Schadstoffkonzentration erreicht. Ferner sind Aktivkohlefilter bekannt und im Einsatz, die eine begrenzte Rückhaltekapazität gegenüber einigen Gasen und Dämpfen und natürlich Stauben und Pollen haben. Größere Konzentrationen können dagegen von diesen Filtern nicht zurückgehalten werden. Auch gibt es filtergängige Gase wie z.B. das giftige Kohlenmonoxid und Feinststäube und Ruße, wie sie z.B. von Dieselmotoren abgegeben werden, die als krebsfördernd gelten.

Die bekannten Ionisationsapparate verfügen in der Regel über einen handbetätigten Umschalter, mit welchem Abgriffe an Transformatoren geschaltet werden können mit der Wirkung, daß der Ionisationsröhre unterschiedliche Spannungen zugeführt werden, um auf diese Weise die Ionisationsleistung einstellen zu können. Eine derartige Anordnung kann durchaus zufriedenstellend arbeiten, wenn es darum geht, z.B. in einem Kühlhaus die Keimzahl gering zu halten, weil in einem Kühlhaus in der Regel keine stark schwankende Luftbelastung mit sonstigen Luftschadstoffen vorkommt. In fast allen anderen Anwendungen jedoch schwankt die Konzentration der Luftbeimengungen erheblich und durchaus in einem Verhältnis 1:10000.

Ein fest eingestellter Ionisationsapparat kann in diesen Fällen nicht zu einer befriedigenden Lösung führen, weil entweder die Ionisationsleistung unzureichend ist und damit die Gerüche und Keime nicht wirksam bekämpft werden oder aber bei für hohe Schadstoffbelastungen ausreichend hoch eingestellte Ionisationsleistung fallen bei sehr geringer Luftbelastung riechbare und womöglich gefährliche Ozon-Konzentrationen störend auf.

Durch die US-A-4 918 568 ist eine Vorrichtung zur Erzeugung aktiver Sauerstoffionen in der Luft für die Luftverbesserung von Räumen bekannt geworden, die einen Luftionisator mit einem elektrischen Transformator umfaßt, der eine zur Luftionisierung ausreichende elektrische Hochspannung erzeugt. Der Luftionisator ist mit einem Sensor gekoppelt, der den Ladungszustand der Luft feststellt. Abhängig vom Ladungszustand kann dadurch mittels einer elektrischen Steuereinrichtung die Hochspannung und damit die elektrische Leistung des Luftionisators und damit dessen Intensität verändert werden. Durch Verändern der Polarität können sowohl positive als auch negative elektrostatische Ladungen erzeugt werden.

### Technische Aufgabe:

Der Erfindung liegt die Aufgabe zugrunde, einen Apparat zur physikalischen Aufbereitung von Luft, insbesondere von Atemluft, zu schaffen, der imstande ist, entsprechend der Belastung der Luft mit Geruchsstoffen oder Abgasen eine von der Konzentration dieser Geruchsstoffe oder Abgase abhängige Ionisierung der Luft durchzuführen. Eines der wesentlichen technischen Probleme besteht darin, die Ionisationsleistung der genannten Ionisationsapparate so an die Luftbelastung anzupassen, daß einerseits eine wirksame Bekämpfung von Gerüchen und Keimen gegeben ist, andererseits jedoch dabei keine überschießenden Ozonkonzentrationen entstehen sollen.

### Offenbarung der Erfindung und deren Vorteile:

Die Lösung der Aufgabe besteht in einer Vorrichtung zur Erzeugung aktiver Sauerstoffionen in der Luft für die Luftverbesserung, insbesondere von Atemluft, bestehend aus wenigstens einem Luftionisator und einem eine zur Luftionisierung ausreichende elektrische Hochspannung erzeugenden elektrischen Transformator, mit einem den Gehalt der Luft an oxidierbaren Gasen feststellenden Luftgütesensor und einer elektrischen Steuereinrichtung, die aufgrund des ermittelten Gehaltes oxidierbarer Gase die dem Luftionisator zugeführte elektrische Energie derart verändert, daß bei niedrigen Konzentrationen oxidierbarer Gase eine nur geringe Ionisationsleistung erfolgt, welche sensorgesteuert und automatisch mit zunehmenden Konzentrationen an oxidierbaren Gasen auf einen Maximumwert steigerbar ist.

In weiterer Ausgestaltung der Erfindung detektiert ein nach dem Luftionisator angeordneter Ionenzähler die Anzahl der in der Luft befindlichen Ionen und wirkt über eine elektronische Schaltung so auf die Vorrichtung bzw. den Luftionisator ein, daß sensorgesteuert und automatisch, vorzugsweise stetig, bei geringer Ionenzahl die Ionisationsleistung des Luftionisators erhöht und bei erhöhter Ionenzahl verringert wird. Damit ist eine Steuerung der Ionisationsleistung des Luftionisators entsprechend der Gasbelastung der Luft, insbesondere von Atemluft, verbunden.

Zur Änderung der Ansteuerleistung des Luftionisators weist der Transformator verschiedene Wicklungsanzapfungen auf und ist über dieselben so ansteuerbar, daß sich wirkungsrichtig und sensorgesteuert eine höhere oder niedrige Betriebsspannung des Luftionisators ergibt. Ebenso kann zur Änderung der Ansteuerleistung des Luftionisators demselben eine Kette von kapazitiven oder ohmschen Widerständen vorgeschaltet sein, die durch geeignete Schaltglieder wirkungsrichtig so überbrückt werden, daß sich entsprechend der Überbrückung eine angepaßte, gesteuerte Ionisationsleistung des Luftionisators ergibt. Die wirkungsrichtige und situationsangepaßte Änderung der Ionisationsleistung kann auch erreicht werden, indem eine Vielzahl von Luftionisatoren vorhanden sind und betrieben werden, wobei die aktive Fläche der betriebenen Luftionisatoren durch geeignete elektrische Schaltglieder den durch den bzw. die Luftgütesensoren ermittelten Erfordernissen angepaßt wird. Die Anhebung der Ionisationsleistung erfolgt sensorgesteuert jeweils dann, wenn die Änderung der vom Luftgütesensor detektierten Gaskonzentration einen bestimmten Quotienten über die Zeit aufweist. Oder die Anhebung der Ionisationsleistung des Luftionisators kann sensorgesteuert jeweils auch dann erfolgen, wenn der gasabhängige Wert des Luftgütesensors oder der Quotient aus der Wertänderung des Luftgütesensors über die Zeit einen bestimmten Wert überschritten hat.

In einer weiteren Ausgestaltung ist die Vorrichtung bzw. der Luftionisator einem Luftbefeuchter vorgeschaltet oder in denselben integriert.

Zur Erweiterung des Einsatzspektrums ist dem Luftionisator ein Ozonsensor nachgeschaltet, welcher mit der elektrischen Steuerschaltung verbunden ist und welcher bei Feststellung eines bestimmten Ozongehaltes in der Luft auf die elektrische Steuerschaltung einwirkt, die die dem Luftionisator zugeführte elektrische Energie verringert. Zur Steuerung der dem Luftionisator zugeführten elektrische Energie beim Auftreten von Ozon kann eine Ansteuerung mit einer sägezahnförmigen Spannung erfolgen, die bei Erreichen einer die Ozonproduktion zulassenden Spannung auf eine solche Spannung zurückgeführt wird, bei welcher einerseits sicher ionisiert, andererseits aber noch nicht ozonisiert wird. Diese sägezahnförmige Spannung kann innerhalb eines Spannungsbandes zwischen den beiden Spannungspegeln rampenförmig oder sägezahnförmig hin und her pendeln.

Vorteilhaft besteht der Luftionisator aus zwei oder mehreren elektrisch leitenden, flächigen oder plattenförmigen Strukturkörpern als Elektroden, die sich planparallel gegenüberstehen und die durch ein Dielektrikum elektrisch voneinander getrennt sind und einen flächigen Kondensator bilden, wobei an die Elektroden eine zur Ionisation von Luft ausreichend hohe Wechselspannung gelegt ist.

Des Weiteren kann der Luftionisator aus einer flächigen äußeren und einer flächigen inneren Elektrode bestehen, die hermetisch von einem die äußere Elektrode tragendes Dielektrikum eingeschlossen ist, wobei die äußere und innere Elektrode je durch einen elektrischen Anschluß kontaktiert sind, und an die innere und die äußere Elektrode eine zur Ionisation von Luft ausreichend hohe elektrische Wechselspannung angeschlossen ist.

Eine Mehrzahl von flachen Luftionisatoren (Flachionisator) sind zu einem Stapel oder zu mehreren elektrisch voneinander isolierten Stapeln geschichtet, die von der zu behandelnden Luft durchströmt werden, wobei an die einzelnen Elektroden eine zur Ionisation von Luft ausreichend hohe elektrische Wechselspannung angelegt wird. Die inneren Elektroden können das gleiche elektrische Potential wie die benachbarten Flachionisatoren haben oder die inneren Elektroden können ein den benachbarten Flachionisatoren ungleiches elektrisches Potential aufweisen.

In vorteilhafter Ausgestaltung ist der Luftionisator luftdurchlässig, wobei die beiden flächigen Elektroden Durchbrechungen aufweisen und gitterartig oder lochartig strukturiert sind und die zu ionisierende Luft den freien Querschnitt der Elektroden durchströmt. Eine der Elektroden besteht aus einem elektrisch leitfähigen Filtermaterial, das von der anderen Elektrode gitterartig, elektrisch isoliert umgeben ist; die zu behandelnde Luft durchströmt beide Elektroden.

Mindestens eine der Elektroden weist eine Vielzahl von gegen die Gegenelektrode gerichteten Nadeln oder Spitzen auf, um den Koronaeffekt zu erhöhen.

Ein Stapel aus Ionisationsplatten ist in einem Belüftungskanal angeordnet und füllt den gesamten Querschnitt des Belüftungskanals aus und bildet dergestalt einen Luftionisator, wobei die Ionisationsplatten entweder mit der schmalseitigen Stirnfläche oder mit ihrer luftdurchströmbaren Querschnittsfläche gegen die Luftströmung hin ausgerichtet sind. Eine solche Vorrichtung kann in einem Außenluft fördernden Luftkanal eines Kraftfahrzeuges eingebaut sein.

### Kurzbezeichnung der Zeichnung:

- Figur 1: einen bekannten Luftionisationsapparat, bestehend aus einer Glasröhre, deren Innen- und Außenwandung mit Drahtgewebe elektrisch leitend ausgelegt ist und die einen Kondensator bildet
- Figur 2: ein Schaubild der von einem Metalloxid-Halbleiter-Sensor zu detektierenden Belastung der Luft mit oxidierbaren Schadstoffen
- Figur 3: eine erfindungsgemäße Vorrichtung zur Detektierung der Luftgüte
- Figur 4: eine erfindungsgemäße Vorrichtung in Kombination mit einem Luftbefeuchter
- Figur 5: eine weitere erfindungsgemäße Vorrichtung in einer elektrischen Schaltung, welche für eine vorgebbare Zeit die Ionisationsleistung erhöht und dann die Ionisationsleistung wieder zurücknimmt oder abschaltet
- Figur 6: eine Schaltung mit einem dem Luftionisator nachgeschalteten Ionendetektor zur Steuerung des Luftionisators
- Figur 7: ein Luftionisator in flacher Bauform als Flachionisator
- Figur 8: eine äußere Struktur des Luftionisators der Figur 7
- Figur 9: ein Plattenstapel aus einer Mehrzahl von erfindungsgemäßen Luftionisatoren (Fig. 7), welche nebeneinander angeordnet sind,
- Figur 10: eine Beschaltungsvariante von zwei Flachionisatoren der Figur 7
- Figur 11: eine weitere Beschaltungsvariante von zwei Flachionisatoren der Figur 7
- Figur 12: ein Flach- oder Plattenionisator, dessen Elektroden gitterförmig luftdurchlässig gestaltet sind und durch deren freien Querschnitt die Luft hindurchströmt
- Figur 13: eine einzelne Elektrode eines derartigen Flach- oder Plattenionisator der Figur 12
- Figur 14: ein weiterer Luftionisator, bei dem die eine, innere luftdurchströmte Elektrode aus Aktivkohle besteht
- Figur 15: eine perspektivische Ansicht eines derartigen Luftionisators gemäß den Figuren 12, 13 und 14, wie er im Ansaugkanal eines Fahrzeugs eingebaut werden kann
- Figur 16: eine Variante eines derartigen Luftionisators, bei welchem die Elektroden einander zugewandte Nadeln oder Spitzen aufweisen,
- Figur 17: eine Anordnung eines Luftionisators in einem Luftkanal, dem ein Ozonsensor zur Steuerung des Luftionisators nachgeschaltet ist,
- Figur 18: ein Schaubild der rampenförmigen Spannung, welche zwischen zwei Spannungswerten VI und VO₃ hin- und herpendelt und
- Figur 20: ein Blockschaltbild einer elektrischen Schaltung zur Erzeugung der rampenförmigen Spannung.

### Wege zur Ausführung der Erfindung:

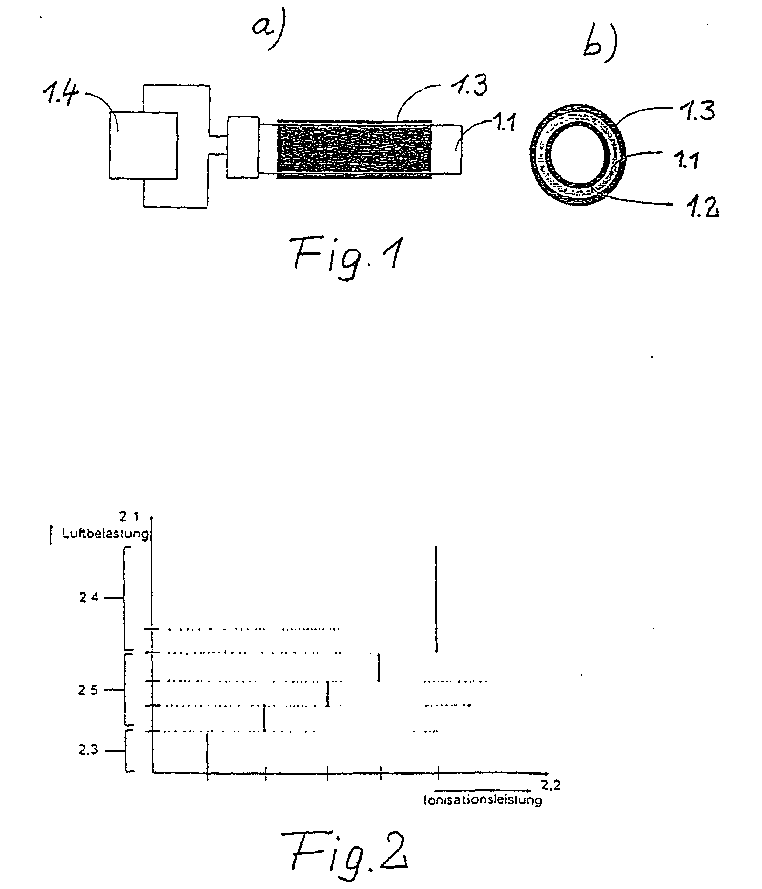

Die Figuren 1a und 1b zeigen zur Erläuterung einen bekannten Ionisationsapparat, welcher im wesentlichen aus einer Glasröhre 1.1 besteht, deren Wand innen und außen mit je einem Drahtgewebe 1.2 bzw. 1.3 elektrisch leitend gelegt ist. An die beiden voneinander isolierten Drahtgewebe ist eine Hochspannung angeschlossen, welche von einem Transformator 1.4 erzeugt wird. Physikalisch handelt es sich hierbei um einen Kondensator, bei dem die Kondensatorflächen durch die beiden Drahtgewebe 1.2 und 1.3 gebildet werden und die Glaswandung 1.1 das Dielektrikum darstellt. Wird an die Drahtgewebe 1.2 und 1.3 eine elektrische Wechselspannung von z.B. 3000 Volt aus dem Transformator 1.4 angelegt, so kommt es zu einer sogenannten "stillen Entladung". Dabei entstehen an den Oberflächen der Drahtgewebe 1.2 und 1.3 negative Ionen von Sauerstoffclustern, das ist sogenannter aktiver Sauerstoff. Bei höheren Spannungen entsteht zwangsläufig als Beiprodukt auch Ozon (O₃), welches einen stechenden Geruch aufweist und bereits in Konzentrationen von nur ca. 50 ppb riechbar ist und in höheren Konzentrationen ab 100 ppb gesundheitsschädlich sein kann. Bekannt und in zahlreichen wissenschaftlichen Untersuchungen nachgewiesen ist die Tatsache, daß der von Ionisationsapparaten produzierte aktive Sauerstoff die Mehrzahl der in der Luft vorkommenden Belastungen mit Geruchsstoff, Keimen, Bakterien und sonstigen Schadstoffen oxidieren und damit unschädlich machen kann. Auch wird die einseitige statische Aufladung von Gerätschaften oder Personen vermieden. Diese bemerkenswerten Eigenschaften sind begründet in der Tatsache, daß die genannten Luftinhaltsstoffe fast durchweg chemisch oxidierbare Stoffe und meist organischer Natur sind.

Wissenschaftliche Untersuchungen, z.B. in Fabriken für Lebensmittel haben gezeigt, daß bei Einsatz von Ionisationsapparaten die Anzahl der Keime/m³ sich von ursprünglich 800-1000 auf 30-60 durchschnittlich vermindert und die Vermehrung der Keime stark behindert ist. Ein praktischer Versuch in einem Flughafen-Restaurant hat ergeben, daß der vorher von Gästen und Personal sehr stark kritisierte Kerosingeruch durch den Einsatz von Ionisationsapparaten in der Luftzufuhr so erfolgreich abgebaut werden konnte, daß er von Testpersonen nicht mehr wahrgenommen werden konnte.

Die Erfindung fußt auf einer sensorgesteuerten Steuerung eines Ionisationsapparates oder Luftionisators entsprechend dem Schaubild der Figur 2. Auf der y-Achse 2.1 ist die von einem Metalloxid-Halbleiter-Sensor, beispielsweise gemäß der WO 97/41423, detektierte Belastung der Luft mit oxidierbaren Schadstoffen aufgetragen, z.B. Zigarettenrauch, Kerosin oder Industrie- oder Kfz-Abgase, Lösungsmittel u.s.w.. Auf der x-Achse 2.2 ist die wünschenswerte Ionisationsleistung aufgetragen. Es ist erkennbar, daß bei sehr geringen Luftbelastungen eine konstant niedrige Ionisationsleistung eingestellt zu werden braucht, um die Zahl der Luftionen, nämlich aktive Sauerstoffionen, auf einem stabilen Niveau zu halten, und um damit dem subjektiv erkennbaren "Geruch" sogenannter "toter Luft" entgegenzuwirken und um die Luft im Gegenteil "frisch" zu machen. Das ist von Bedeutung, weil Menschen zwar keine aktiven Luftionen tatsächlich riechen können, aber den Unterschied zwischen von außenkommender, mit einer hohen Zahl aktiver Luftionen versehener Luft und "abgestandener Luft" in abgeschlossenen Innenräumen ohne Anteile aktiver Luftionen sicher feststellen können. Bei hohen Luftbelastungen 2.3 stagniert die Ionisationsleistung aufgrund der in der Praxis begrenzten Leistung der Ionisationsapparate. Zwischen diesen Punkten folgt automatisch und sensorgesteuert, vorzugsweise stetig, die Ionisationsleistung 2.4 und 2.5 der Luftbelastung gemäß der Erfindung.

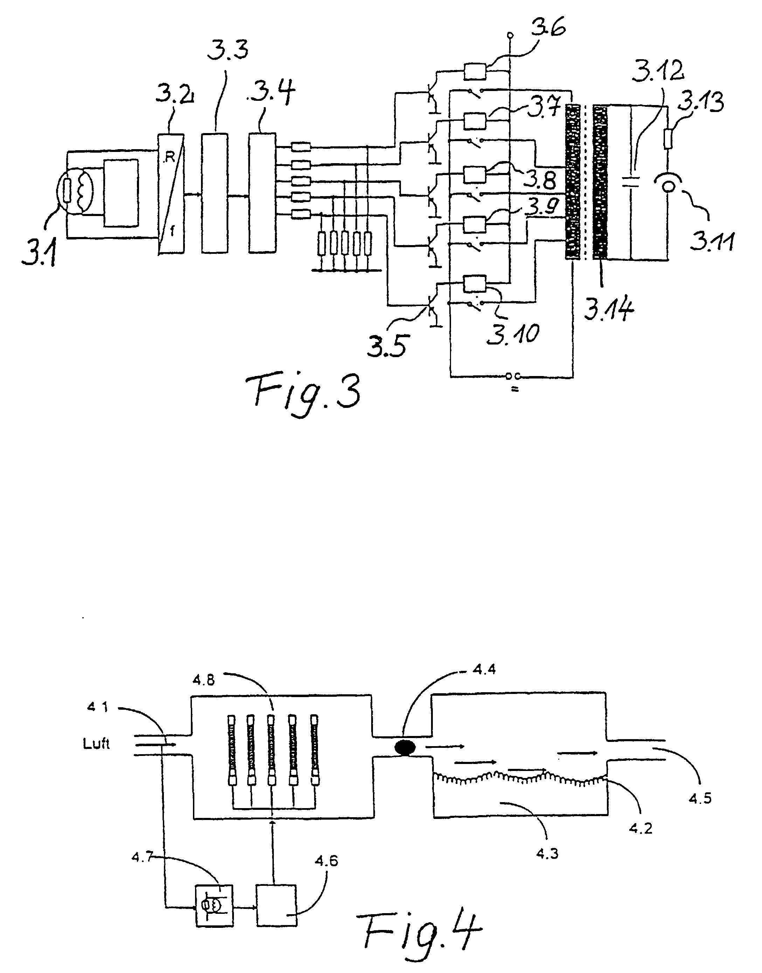

Ein Sensor zur Detektierung der Luftgüte ist in Figur 3 dargestellt. Ein beheiztes Sensorelement 3.1, nämlich z.B. ein Metalloxid-Halbleiter-Sensor nach der WO 97/41423, wirkt wie ein elektrischer Widerstand, der bei unbelasteter Luft einen typischen Wert von ca. 40 kOhm annimmt. Sind dagegen in der Luft oxidierbare Gase oder Dämpfe vorhanden, so sinkt der elektrische Widerstand auf einen der Gaskonzentration entsprechenden Wert von z.B. 5 kOhm oder weniger. Diese Widerstandsänderung ist somit ein Maß für die Belastung der Luft mit Schadstoffen. Im einfachsten Fall kann der Sensor 3.1 mit einem Ohm'schen Widerstand in Reihe geschaltet werden, so daß sich ein Spannungsteiler ergibt. Dann ist die Spannungsänderung ein Maß für die Belastung der Luft.

Des Weiteren ist vorgeschlagen worden, den Ionisationsapparat mit Impulspaketen anzusteuern, wobei dann das Impuls-Pausen-Verhältnis sensorgesteuert wird. Bei sehr belasteter Luft wird bei diesem Verfahren permanent ionisiert. Bei gering belasteter Luft wird ein Impulspaket nur geringer Dauer erzeugt, welches von einer vergleichsweise längeren Pause gefolgt wird. Das Impuls-Pausen-Verhältnis ist insofern eine Funktion der Luftgüte. Nachteilig ist bei ansonsten einwandfreier Funktion, daß die bei der Ionisation sich zwangsläufig ergebenden prasselnden oder brodelnden Geräusche des Ionisationsapparates akustisch sozusagen zerhackt werden, als Ergebnis mäßig an das Geräusch einer Grille erinnert und von Personen in der Regel als sehr lästig empfunden wird.

Es ist des Weiteren vorgeschlagen worden, nach dem bekannten Verfahren der Phasenanschnittsteuerung die der Ionisationsapparatur zugeführte Leistung so zu beeinflussen, daß sich eine auf einer Kennlinie verlaufende Steuermöglichkeit ergibt. Dieses Verfahren erweist sich in der Praxis als schwierig anwendbar, weil die anzusteuernde induktive Last erhebliche Toleranzen aufweist und eine einwandfreie und reproduzierbare Steuerung der Ionisationsleistung einschränkt. Im übrigen treten bei dieser Form der Ansteuerung schwer zu beherrschende elektromagnetische Abstrahlungen auf, die nur mit erheblichem Entstöraufwand zu unterdrücken sind.

Die in Figur 3 gezeigte Schaltung des Sensors geht folgenden Weg, indem der Widerstand des Sensorelements 3.1 in eine elektrische Schwingschaltung 3.2 eingebaut und Teil dieser elektrischen Schwingschaltung 3.2 ist. Es ergibt sich eine Frequenz des Schwingkreises, deren Höhe eine Funktion des Sensorwiderstandes ist. Bei unbelasteter Luft ergibt sich typisch eine niedrige, bei belasteter Luft typisch eine deutlich höhere Frequenz.

Ein der Schwingschaltung 3.2 nachgeschalteter elektrischer Frequenzzähler 3.3 stellt die Frequenz fest und schaltet entsprechend seine Ausgangskontakte, die bevorzugt eine binäre Codierung der momentanen ausgegebenen Frequenz ausgeben. Diese Signale werden auf eine Auswertelogik 3.4 gegeben, die die Signale decodiert und damit Treibertransistoren 3.5 für bestimmte Frequenzgruppen oder Frequenzabstufungen analog Luftgüte/Pegel ansteuert, welche nachgeschaltete Relais 3.6, 3.7, 3.8, 3.9 und 3.10 schalten. Die Relais 3.6 bis 3.10 schalten ihrerseits einen elektrischen Transformator 3.14 dergestalt, daß die erzeugte Sekundärwechselspannung abgestuft, z.B. zwischen 1500 bis 3200 Volt, an einer Ionisationsröhre oder einem Luftionisator 3.11 angelegt wird. Eine Kombination aus einem der Sekundärwicklung des Transformators 3.14 parallel geschaltetem Kondensator 3.12 und einem ebenfalls der Sekundärwicklung parallel geschaltetem Widerstand 3.13 dienen der Unterdrückung von hochfrequenten Störimpulsen, welche aufgrund der Entladungsvorgänge in der Ionisationsröhre entstehen, falls eine solche verwendet wird.

Es sind andere praktische Schaltungen denkbar, insbesondere analoge Schaltungen mit den Ionisationsstufen zugeordneten Fensterdiskriminatoren oder voll digitale Lösungen mit Mikroprozessor. Allen Schaltungen jedoch ist erfindungsgemäß gemeinsam, daß die Ionisationsleistung durch Ändern der zugeführten elektrischen Energie in Abhängigkeit von einem Luftgütesensor automatisch gesteuert wird. Weiterhin können die elektrischen oder mechanischen Relais durch Halbleiterschalter, z.B. durch Triacs, ersetzt werden, ohne daß der Grundgedanken der Erfindung dadurch verlassen wird.

Dadurch wird erreicht, daß stets eine ausreichende und eine angepaßte Ionisationsleistung aktiver Ionen soweit erzeugt wird, daß eine wirksame Bekämpfung von Gerüchen, organischen Substanzen, Bakterien und Viren gewährleistet ist und daß statische Aufladung erheblich zurückgedrängt werden, was bei Einsatz z.B. in der Elektroproduktion von wesentlicher Bedeutung ist.

Gleichzeitig wird gewährleistet, daß niemals Belästigungen oder gar Gefährdungen von Personen durch überschießende Ionisationsleistungen vorkommen, womit riechbare Ozonkonzentrationen verbunden sein könnten. Damit ist der effektive und gefahrlose Einsatz der vorteilhaften Ionisationstechnik überall dort möglich, wo gefährliche oder unangenehme Luftinhaltsstoffe oder Keime bekämpft werden sollen und wo ein elektrisch einwandfreies Raumklima statischen Aufladungen vorbeugen soll.

Des Weiteren kann diese Technik erfindungsgemäß mit bekannten Luftbehandlungsanlagen kombiniert werden. Es ist z.B. bekannt, die Luft apperativ zu befeuchten, indem z.B. eine Batterie von etwa zur Hälfte in Wasser sich befindenden drehbaren Scheiben von Luft durchströmt wird. Dabei wird Wasser auf den relativ großen Flächen der Scheibenbatterie verdunstet, wobei gleichzeitig Stäube und manche wasserlöslichen Luftbestandteile in Wasser gebunden werden. Nachteilig ist jedoch, daß Keime im feuchten Milieu der nassen Platten geradezu gezüchtet werden, und im Wasserbad erhebliche und gefährliche Konzentrationen erreichen können. Bei der Beströmung der Platten können dann Keime beim Verdampfen des Wassers mitgerissen werden und sich anschließend nachteilig in der Atemluft anreichern.

Aus diesem Grund wird den Anwendern derartiger Apparate empfohlen, dem Wasserbad sterilisierende Zusätze zuzugeben, was zusätzliche Kosten und Mühen macht und auch biologisch bedenklich ist, weil ja auch diese Zusätze zum Teil luftgängig sind und in die Atemluft verdunsten.

In Figur 4 ist eine Kombination der Vorrichtung gemäß Figur 3 mit einer Befeuchtungstechnik gezeigt. An der Einströmseite 4.1 der Vorrichtung gemäß Figur 4 für die Luft wird diese über einen oder mehrere Luftionisatoren 4.8 geführt. Die Ionisationsleistung wird durch ein elektrisches Steuergerät 4.6 in Abhängigkeit von einem Gassensor 4.7 zur Detektion oxidierbarer Luftbestandteile gesteuert, gemäß den Ausführungen zu Figur 3.

Durch die Ionisation und die so erzeugten aktiven Sauerstoffionen werden Keime zuverlässig vernichtet und Gerüche und gas- und dampfförmige, oxidierbare Luftbestandteile oxidiert und damit unschädlich gemacht. Ein Teil der aktiven Sauerstoffionen wird im Wasser eines Wasserbades 4.3 an der Befeuchtungsoberfläche 4.2 gebunden und gelangt somit in das Wasserbad 4.3 und kann dort vorhandene Keime bekämpfen und verhindern, daß sich diese weiterhin vermehren können. Der Lufttransport wird durch einen Ventilator 4.4 gewährleistet. An der Ausblasseite 4.5 nach dem Wasserbad 4.3 wird Luft zur Verfügung gestellt, deren Qualität durch die erfindungsgemäße Anordnung erheblich gesteigert ist. Die Luft ist einerseits angenehm befeuchtet und andererseits von gas- und dampfförmigen Schad- und Geruchsstoffen und von Keimen befreit.

Ein weiteres Anwendungsgebiet sind Ionisationsapparate zur Luftauffrischung in Innenräumen mit eingeschränkter Ventilation, z.B. in Toiletten, Dusch- und Baderäumen oder auch in Lager- und Kellerräumen. Hier kann mit einer erfindungsgemäßen kleinen Ionisationsapparatur permanent mit geringer Leistung so gefahren werden, daß Keime vernichtet werden und die Luft stets angenehm frisch ist, aufgrund der Anwesenheit aktiver Sauerstoffionen.

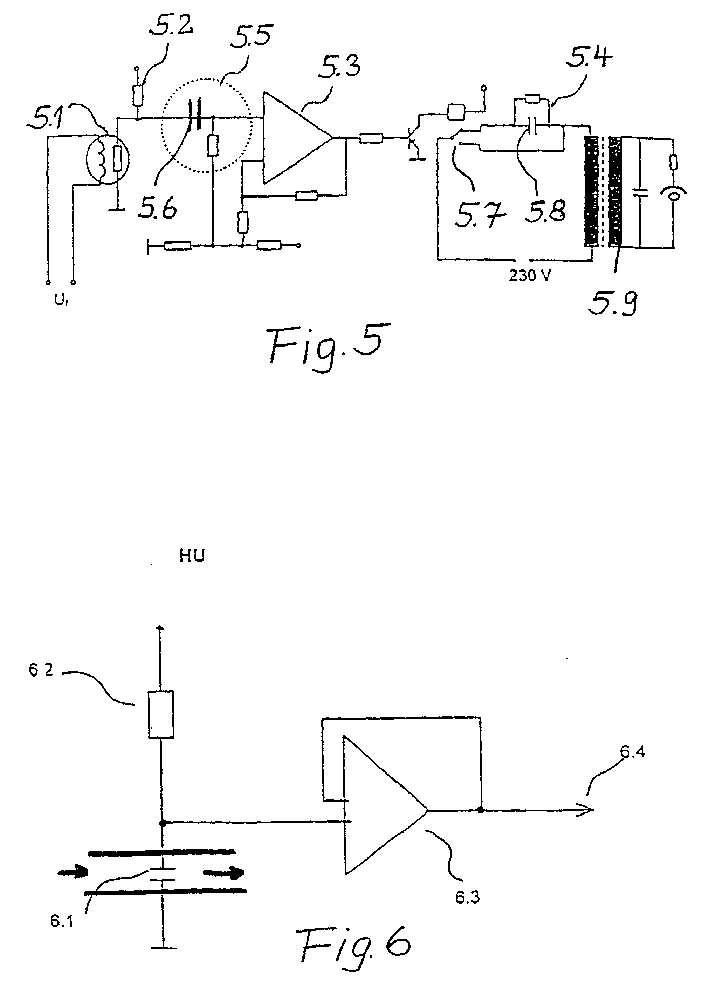

Kommt es zu einer Anreicherung der Luft mit gasförmigen Stoffen, z.B. bei Benutzung der Toilette oder bei Freisetzung von Geruchsstoffen durch neue Einlagerungen in den Raum, so wird dies gemäß Figur 5 von einem Sensor 5.1 detektiert, welcher wie zur Figur 3 beschrieben arbeitet, und durch entsprechende Beschaltung, wie zu Figur 3 beschrieben, kann die Ionisationsleistung bedarfsgerecht erhöht werden. Erfindungsgemäß wird hier nur für eine einstellbare, begrenzte Zeit die Ionisationsleistung erhöht, wenn nämlich der Gassensor eine Erhöhung der Luftbelastung detektiert.

Dazu ist der Gassensor 5.1 gemäß Figur 5 mit einem elektrischen Widerstand 5.2 zu einem Spannungsteiler zusammengeschaltet. Die sich am Widerstand 5.2 ergebende elektrische Teilerspannung ist dann eine Funktion der Luftqualität. Wird das Sensorelement 5.1 aufgrund einer ansteigenden Luftbelastung niederohmisch, ändert sich die Teilerspannung am Widerstand 5.2 entsprechend der Spannungsteilerregel. Ein als Hochpaß 5.5 mit angepaßter Übertragungsfrequenz nachgeschalteter Kondensator 5.6 überträgt nur die Spannungsänderung und nicht den statischen Sensorpegel auf einen Komparator 5.3, dessen Ausgangsimpuls ein Zeitglied 5.4 triggert. Der Ausgang des Zeitgliedes 5.4 steuert ein Umschaltrelais 5.7 an. Während im Normalfall eine verringerte Ionisationsleistung durch Vorschaltung eines Kondensators 5.8 vor dem Transformator 5.9 eingeschaltet ist, wirkt dann, wenn die Luftbelastung um einen vorgegebenen Betrag steigt, vorübergehend eine höhere Ionisationsleistung mittels des Umschaltrelais 5.7 eingeschaltet, indem der Transformator 5.9 direkt mit Netzspannung angesteuert wird.

Die Anzahl der in der Luft vorhandenen negativen Ionen ist bestimmend für das elektrische Raumklima. Wird Raumluft "abgestanden" oder mit Partikeln, Dämpfen oder Rauch belastet, sinkt die Anzahl der Ionen teilweise auf Null. Bei Behandlung der Luft mit elektrischen Luftionisatoren, wie vorstehend beschrieben, steigt die Zahl an und erreicht, daß in frischer Außenluft "übliche Niveau".

Bei übertriebener Ionisierung kommt es allerdings zur Bildung von riechbarem Ozon, was vermieden werden sollte.

Deshalb wird erfindungsgemäß vorgeschlagen, dem Luftionisator einen Ionendetektor nachzuschalten oder den Ionendetektor in dem zu belüfteten Raum anzubringen. Gemäß der Figur 6 besteht ein derartiger Ionendetektor im wesentlichen aus einem Plattenkondensator 6.1 mit dem Dielektrikum Luft. Eine bestimmte Menge Luft wird von einem Kleinventilator durch die Platten des Plattenkondensators 6.1 gedrückt. Die Platten des Plattenkondensators 6.1 stehen unter elektrischer Ladung, wobei eine elektrische Spannung durch einen Widerstand 6.2 an die Platten 6.1 gelegt ist. Ionen erzeugen einen Ladungstransport und insofern einen geringen Stromfluß, der am Widerstand 6.2 abgegriffen werden kann und der als Eingangssignal einem Impedanzwandler 6.3 zugeführt wird und als Signal 6.4 vom Impedanzwandler 6.3 ausgegeben wird. Mit diesem Signal kann beispielsweise die Sensoranordnung gemäß Figur 3 ersetzt werden. Erfindungsgemäß kann nun das die Ionisation repräsentierende Signal 6.4 als Steuerungssignal derart eingesetzt werden, daß die Ionisationsleistung der Apparatur erhöht wird, wenn wenig Ionen vorhanden sind und daß die Ionisationsleistung abgesenkt wird, wenn die gewünschte Ionenzahl erreicht oder überschritten ist.

Erfindungsgemäß steuern somit die Ionendetektoren gemäß Figur 6 die Ionisationsleistung durch elektrischen Einfluß nach einer der vorstehend beschriebenen Methoden; alternativ ist die Steuerung mit konstanter Spannung bei gesteuertem Impuls-Pausen-Verhältnissen möglich; ebenso ist alternativ die Ansteuerung mit konstanter Spannung möglich, wobei die Energiezuführung in den einzelnen Wechselspannungszyklen mit der sogenannten Phasenanschnittsteuerung erfolgen kann.

Eine weitere Möglichkeit, die Ionisationsleistung zu verändern, besteht darin, die eingesetzte aktive Fläche der Ionisationsapparate z.B. durch Verändern der Anzahl der eingesetzten Ionisationsröhren oder Ionisationseinrichtungen zu verändern. Insbesondere bei großen Anlagen mit erheblicher Luftleistung werden in der Regel Anordnungen mit einer Vielzahl von Luftionisatoren eingesetzt. Erfindungsgemäß wird vorgeschlagen, eine der vorstehend erwähnten Ionisationsapparate mit einer geeigneten Steuerelektronik so einzusetzen, daß in Abhängigkeit von der gewünschten Ionisations-Intensität die Anzahl der aktiv betriebenen Ionisationsapparate oder Luftionisatoren verändert wird. Allgemein kann dazu gesagt werden, daß bei hoher Luftverschmutzung oder hoher Luftmenge alle Ionisationsapparate betrieben und bei sauberer Luft nur ein einziger oder kein Ionisationsapparat betrieben wird, um überschießende Ionenproduktion und/oder die Gefahr der Ozonisierung zu vermeiden.

Es ist ein wesentlicher Teil der Erfindung, die bis heute vielfach genutzten und vorstehend in Figur 1 genannten röhrenförmigen Luftionisatoren durch andere vorteilhafte Bauformen zu ersetzen.

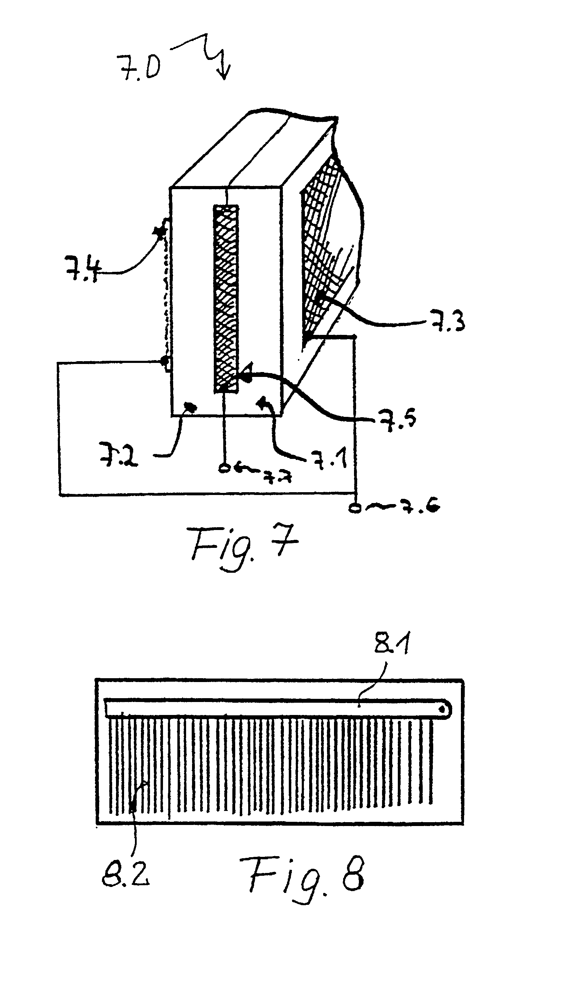

In Figur 7 ist ein erfindungsgemäßer Luftionisator 7.0 gezeigt, der sich durch ein günstigeres Verhältnis zwischen Volumen und Oberfläche auszeichnet als die bekannten Glasröhrenionisatoren des Standes der Technik. Da die Stirnflächen klein sind, ist der Strömungswiderstand der erfindungsgemäßen Flachionisatoren erheblich geringer als bei den bekannten Röhren. Hinsichtlich des Aufbaus ist eine innere elektrisch leitende Fläche 7.5 von zwei Scheiben 7.1 und 7.2 oder Platten aus elektrisch isolierendem Material hermetisch eingeschlossen. Es kommen dabei Materialien, wie Glas, Keramik, spezielle Kunststoffe wie PTFE oder ähnliche Materialien in Frage, welche eine hohe elektrische Durchschlagsfestigkeit aufweisen und ein gutes Dielektrikum bilden. Die Scheiben 7.1 und 7.2 weisen äußerlich elektrisch leitende Strukturen 7.3 und 7.4 auf. Sowohl die innere elektrisch leitende Fläche 7.5 als auch die äußeren Strukturen 7.3 und 7.4 sind elektrisch leitend kontaktiert, wobei die Zuführung zu den Strukturen 7.3 und 7.4 durch die Zuleitung 7.6 und der elektrische Anschluss für die elektrisch leitende Fläche 7.5 durch die Zuleitung 7.7 gebildet ist. Die äußerlichen Strukturen 7.3 und 7.4 können mit Techniken wie Siebdruck, Bedampfung oder Ätzung bzw. Laserstrukturierung so aufgebaut werden, dass sie eine Vielzahl von Kanten oder Spitzen aufweist und an diesen Kanten oder Spitzen der Struktur hohe elektrische Feldstärkenüberhöhungen auftreten.

Ein Beispiel einer derartigen Gestaltung der Strukturen entsprechend den Strukturen 7.3 und 7.4 ist in Figur 8 gezeigt. Dabei sind an Leiterbahnen 8.1 kammartige oder stacheldrahtartige Fortsätze 8.2 angebracht, die z.B. Stacheldraht nicht unähnlich sehen. So sind Strukturen im genannten Sinn erstellbar, die der Erzeugung von hohen Feldstärken an Kanten oder Spitzen dienen.

An die Zuleitungen 7.6 und 7.7 in Figur 7 wird im Betrieb eine hohe elektrische Wechselspannung angelegt. Dabei wird der sich zwischen innere und äußere Struktur befindliche Kondensator, um den es sich letztlich handelt, laufend umgeladen. Die sich in den einzelnen Phasen an den äußeren Strukturen 7.3 und 7.4 bildenden ionisierten Gasmoleküle werden in der nächsten, umgekehrt gepolten Phase abgestoßen und gelangen so in die Umgebungsluft.

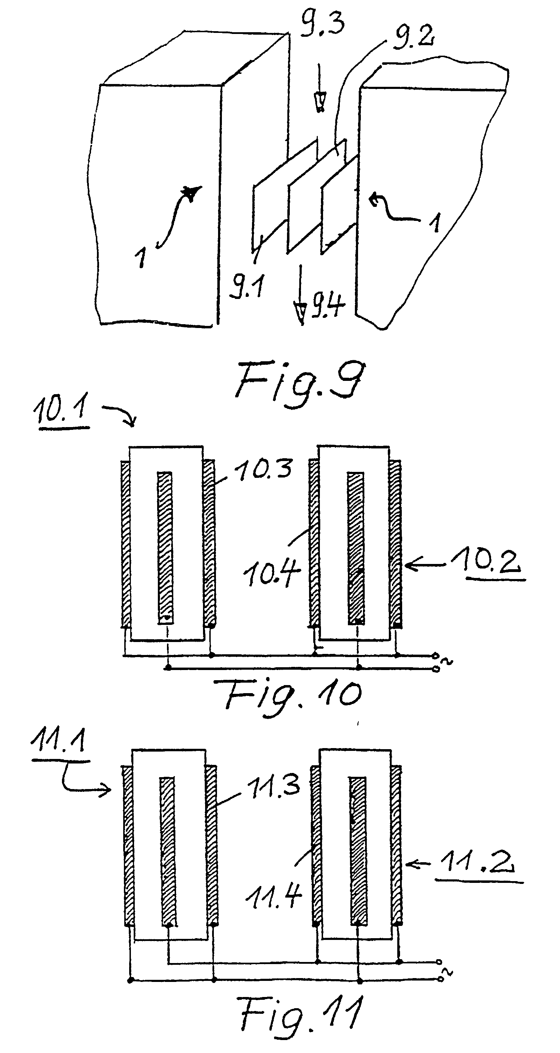

Mit einem derartigen Platten- oder Flachionisator gemäß der Figur 7 lassen sich Plattenstapel gemäß Figur 9 aufbauen. Die einzelnen Platten 9.1, 9.2, welche der Plattenstruktur 7.0 der Figur 7 entsprechen, sind dabei in einem definierten Abstand und isoliert voneinander so angeordnet, dass die aufzubereitende Luft 9.3 durch den Plattenstapel 9.1, 9.2... strömt und über die große Fläche des gesamten Plattenstapels ein Maximum an Ionen aufnehmen kann. Die ausströmende Luft 9.4 ist mit Sauerstoffionen angereichert. Höchst vorteilhaft ist, dass die Stirnfläche des Plattenstapels aus den Platten 9.1, 9.2 ... gegenüber der anströmenden Luft 9.3 vergleichsweise gering ist und dass bei kleinem Volumen eine relativ große Ionisationsfläche aufgrund der Plattenfläche erreicht wird.

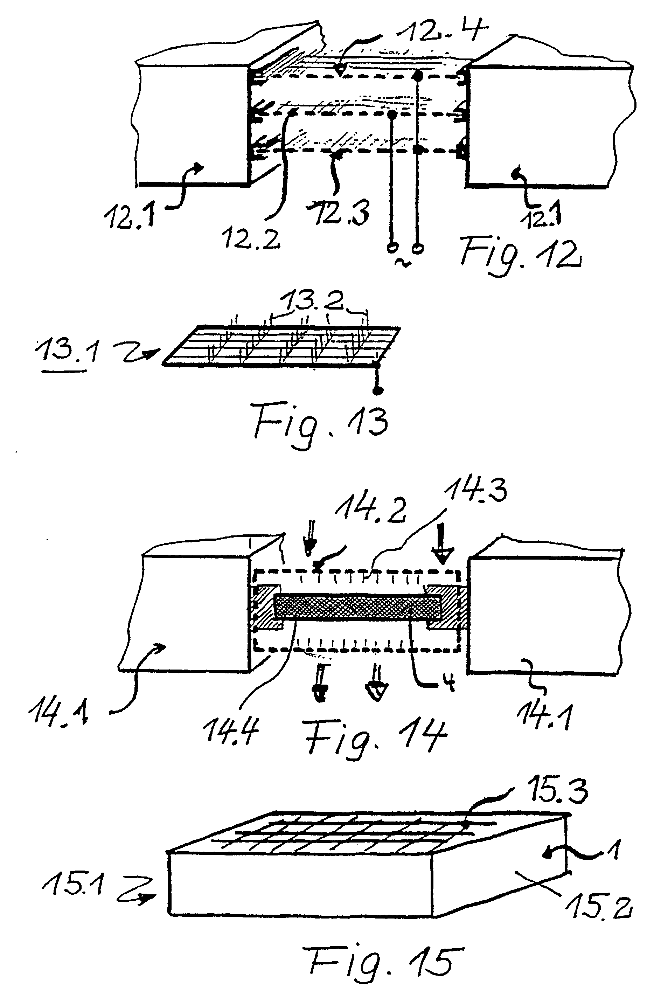

Dabei sind für die Platten 9.1, 9.2... verschiedene Beschaltungsvarianten möglich, die in den Figuren 10 und 11 gezeigt sind. Figur 10 zeigt zwei Plattenionisatoren 10.1 und 10.2 gemäß der Figur 7, bei denen die inneren und äußeren Flächen jeweils das gleiche elektrische Potential haben und insofern parallel geschaltet sind, wie es der Figur 10 zu entnehmen ist. Der Vorteil dieser Schaltungsmethode ist, dass die Abstände zwischen den einzelnen Platten 10.1 und 10.2 sehr gering gewählt werden können, weil ja die sich zugewandten jeweiligen äußeren Flächen 10.3 bzw. 10.4 das gleiche Potential haben und somit keine Gefahr eines elektrischen Überschlages besteht. Auf geringem Bauvolumen kann somit eine extrem große aktive Ionisationsfläche untergebracht werden.

Figur 11 zeigt zwei Plattenionisatoren 11.1 und 11.2, bei denen die inneren und äußeren Flächen abwechselnd wechselndes Potentional in Bezug auf die Nachbarplatten haben und insofern antiparallel geschaltet sind, wobei der Abstand zwischen zwei benachbarten äußeren Flächen 11.3 und 11.4 so gewählt ist, dass sich keine elektrischen Überschläge ereignen. Der Vorteil dieser Schaltung ist, dass die Luftstrecke zwischen den Ionisationsplatten bzw. zwischen den beiden äußeren Flächen 11.3 und 11.4 zweier Luftionisatoren 11.1 und 11.2 ebenfalls als Dielektrikum wirkt und sich zwischen den Platten ein erhebliches elektrisches Feld aufbauen kann, welches insbesondere polare Moleküle, wie z.B. Kohlewasserstoffmoleküle, zu zerreißen im Stande ist. Die Kombination der Einflüsse "Oxidieren durch aktive Sauerstoffionen" und "Zerreißen von polaren Molekülen im elektrischen Wechselfeld" lässt sich die Wirkung von Ionisationsapparaten potenzieren.

Figur 12 zeigt eine weitere erfindungsgemäße, vorteilhafte Bauform eines Luftionisators. Innerhalb eines isolierten bzw. isolierenden Rahmens 12.1, der vorzugsweise einen Strömungskanal für die durchgeleitete Luft bildet, befinden sich gitterähnliche, flache Strukturen 12.2, 12.3, 12.4, welche flächige, gitterähnliche Körper sind und elektrisch leitende Oberflächen aufweisen. Diese Körper 12.2, 12.3, 12.4 sind im Luftstrom planparallel übereinander angeordnet und elektrisch so kontaktiert, dass sie jeweils wechselndes elektrisches Potential aufweisen. Die Abstände sind so gewählt, dass keine elektrischen Überschläge auftreten können. Das Material der gitterähnlichen Flachkörper 12.2, 12.3, 12.4 kann aus Drahtgewebe, aus gestanzten Metallteilen oder ähnlichem, elektrisch leitfähigem Material bestehen.

Die Produktionsrate von Ionen wird erfindungsgemäß durch eine Ausgestaltung von elektrisch leitenden Flachkörpern 13.1 dadurch erhöht, wenn die elektrisch leitenden Flachkörper - oder die Flachkörper 12.2, 12.3, 12.4 in Figur 12 - ähnlich wie Stacheldraht aufgebaut sind und über zahlreiche nadelförmige oder zackenförmige Erhebungen 13.2. verfügen, an denen der Koronaeffekt besonders deutlich auftritt.

Eine weitere Ausgestaltung ist in Figur 14 gezeigt, wobei wiederum in einem elektrisch isolierenden, einen Luftkanal bildenden Rahmen 14.1 eine Elektrode 14.4 angeordnet ist, welche von einem Käfig 14.2 isoliert umschlossen ist. Die innere Elektrode 14.4 ist elektrisch isoliert von der äußeren Elektrode 14.2 aufgehängt und besteht aus einem elektrisch leitfähigen Filtermaterial aus formgepresster und aufgeschäumter Aktivkohle. Die Luftführung ist dergestalt, dass die Luft durch die äußere Elektrode 14.2 strömt und das elektrisch leitfähige Filtermaterial 14.4 ebenfalls durchströmend durchsetzt. Im elektrischen Wechselfeld zwischen den beiden Elektroden 14.2 und 14.4 werden Luftinhaltsstoffe wie beschrieben zerrissen und gleichzeitig von den aktiven Sauerstoffionen aufoxidiert. Stäube und Partikel werden in der Aktivkohlematte 14.4 festgehalten. Impulsartig auftretende Konzentrationen von Gasen oder Dämpfen werden vorübergehend in der Aktivkohlematte gebunden und dort durch die ebenfalls durch die Matte strömenden aktiven Sauerstoffionen oxidiert, so dass die Matte sich stets regeneriert und es nicht zu den gefürchteten Desorptionseffekten kommen kann. Diese Konstruktion kann sich als sogenannte "Sandwich-Struktur"beliebig oft wiederholen.

Die äußere, käfigartige Elektrode 14.2 kann des Weiteren nach innen weisende nadelförmige oder zackenförmige, elektrisch leitende Spitzen 14.3 aufweisen, an denen der Koronaeffekt besonders deutlich auftritt.

Der Ionisationseffekt kann noch erhöht werden, wenn auch in die Aktivkohlematte eine Vielzahl elektrisch kontaktierter Nadeln oder Spitzen eingetrieben wird, um gegenüber der Gegenelektrode 14.2 bzw. den Spitzen 14.3 den die Ionenproduktion potenzierenden Koronaeffekt zu erreichen. Selbstverständlich können die genannten Nadeln auch auf der Gegenelektrode ähnlich wie in Figur 13, ausgebildet sein oder sich auf beiden Flächen gleichermaßen befinden.

Für Fahrzeuge liegt es nahe, eine physikalische Luftbehandlung, wie beschrieben, zur Behandlung der einströmenden Außenluft einzusetzen. Dadurch wird erreicht, dass oxidierbare Gase, wie Benzindämpfe, Kerosin- oder Dieselgerüche giftiges Kohlenmonoxid oder unverbrannte Kohle, Wasserstoffe, Benzol usw. aufoxidiert und insofern unschädlich gemacht werden. Eine solche Luftbehandlungseinrichtung für Fahrzeuge kann in der Nähe des Wärmetauschers des Fahrzeugs angeordnet sein.

Figur 15 zeigt ein derartiges, anschlussfertiges Ionisationsmodul 15.1, jeweils bestehend aus einem äußeren elektrisch isolierenden Rahmen 15.2, welcher gleichzeitig als röhrenartiger Luftleitkanal dient, dessen eine Oberfläche angeströmt wird und an dessen andere Oberfläche die behandelte Luft wieder austritt. Innerhalb des Rahmens spannen sich über die Querschnittsfläche derarige flächige Elektroden 15.3 auf, wie sie vorstehend in den Figuren 7 bis 14 beschrieben sind.

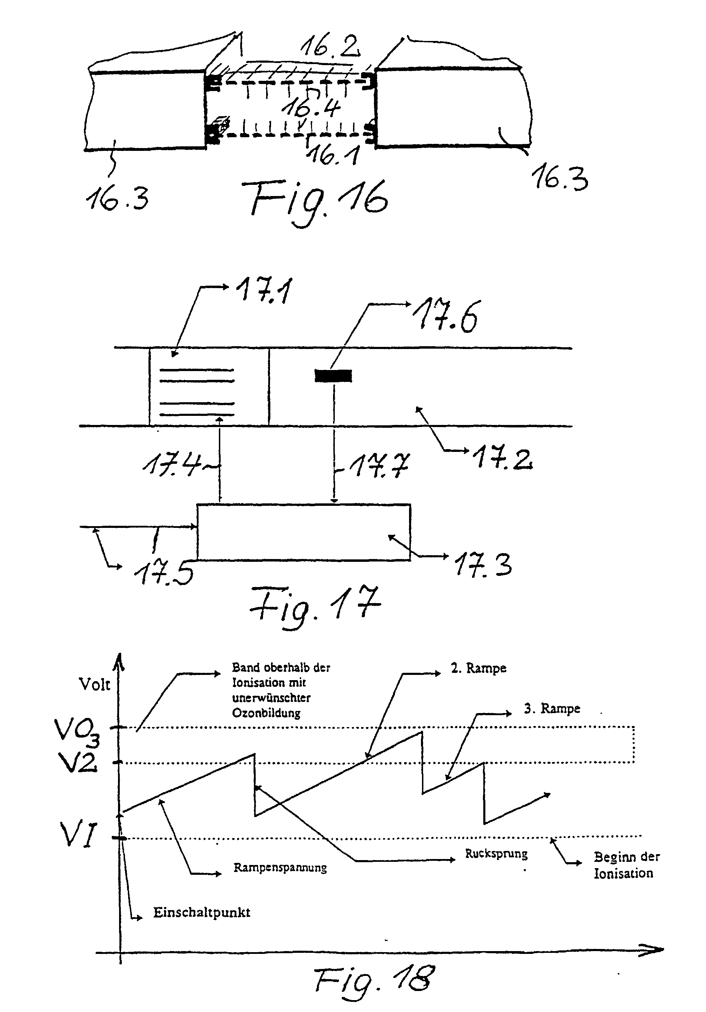

Figur 16 zeigt ein ähnliches Modul, bei dem hier zwei elektrisch leitende flächige luftdurchlässige Platten 16.1 und 16.2 innerhalb eines Rahmens 16.3 gehaltert sind. Beide Elektroden 16.1 und 16.2 weisen nach innen aufeinanderzuragende nadelförmige oder zackenförmige Spitzen 16.4 auf, um den Koronaeffekt zu verstärken.

Figur 17 zeigt eine besonders vorteilhafte Methode zur Steuerung einer Luftionisationseinheit, wobei die Luftionisation entweder durch einen röhrenförmigen oder einen plattenförmigen Luftionisator 17.1 durchgeführt wird, der in einem Luftkanal 17.2 angeordnet ist. Der Luftionisator 17.1 wird über einen Steuerelektronik-Modul 17.3 über die Leitung 17.4 mit Spannung versorgt. Der Steuerelektronik-Modul 17.3 ist an das Netz 17.5 angeschlossen.

In Strömungsrichtung hinter den Luftionisator 17.1 ist ein Ozonsensor 17.6 angeordnet, der möglicherweise im Luftionisator 17.1 erzeugtes Ozon zu detektieren im Stande ist. Der Ozonsensor 17.6 ist über eine Leitung 17.7 mit dem Steuerelektronik-Modul 17.3 verbunden.

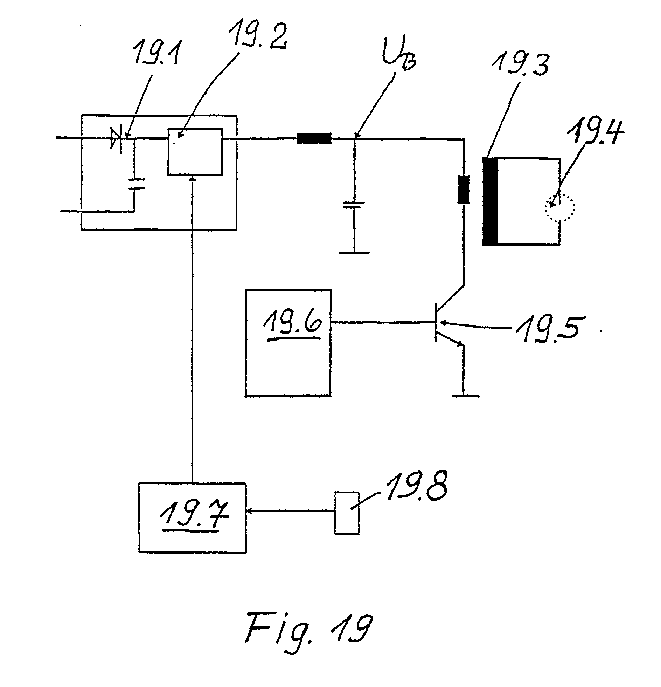

In Figur 18 ist die Funktionsweise der Schaltung von Figur 17 erklärt. Es ist bekannt, dass die Ionisationsfunktion sich oberhalb einer Mindestspannung VI, dem Einschaltpunkt, einstellt. Es ist weiterhin bekannt, dass oberhalb einer weiteren Spannungsgrenze, nämlich die Spannung VO₃, die Entwicklung des unerwünschten Ozons eintritt. Diese Spannungsgrenze liegt mit zunehmender Luftfeuchte und zunehmender Luftverschmutzung höher als bei trockener und sauberer Luft. Zwischen diesen beiden Spannungen VI und VO₃ liegt somit der Spannungs-Arbeitsbereich, den der Luftionisator durchlaufen soll. Deshalb wird eine sägezahnförmige Hochspannung einer Frequenz zwischen 0,05 bis 0,2 Hz erzeugt, welche zwischen dem Einschaltpunkt oberhalb des Beginns der Ionisation VI und der Spannung VO₃ mit der unerwünschten Ozonbildung hin- und herpendelt. Nach dem Einschalten im Einschaltpunkt erhöht sich die Hochspannung in Form einer Rampenspannung stetig um einen festgelegten Betrag pro Zeiteinheit. Oberhalb der Spannung VO₃, deren exakte Höhe von Parametern wie Luftstrom, Luftfeuchte, Luftverschmutzung usw. abhängt, kommt es zur Produktion von Ozon. Der Ozonsensor 17.6 erkennt dies und meldet dies dem Steuerelektronik-Modul 17.3, der die Hochspannung um einen bestimmten Betrag, nämlich den Rücksprung in Figur 18, zurücksetzt, welches sich in dem Band befindet, in welchem ionisiert wird, allerdings noch sicher keine Ozonproduktion stattfindet. Anschließend wird erneut die Hochspannung erhöht und die zweite Rampe durchfahren, bis erneut die Ozonbildung beim Spannungspunkt VO₃ einsetzt und erneut die Hochspannung durch einen Rücksprung von der zweiten Rampe herabgesetzt wird. Anschließend kann dieser Vorgang weiter wiederholt werden. Im Ergebnis fährt somit die Anordnung die Ionisationsanlage derart, dass die Hochspannung immer sich in einem Bereich befindet, in welchem einerseits sicher ionisiert wird, aber andererseits der Bereich, in welchem Ozonbildung stattfindet, zuverlässig vermieden wird. Vorteilhaft wird durch die Erfindung die Ionisierungsspannung für den Luftionisator 17.1 stets so geführt, dass eine maximale Ionisation oder eben eine gewünschte Ionisation stattfindet, ohne jedoch eine Überproduktion von Ozon zuzulassen.

In Figur 19 ist ein Prinzipschaltbild einer elektrischen Schaltung geschrieben, wie die Funktion der Figur 18 elektrisch erfüllen kann. In einen Netzgleichrichter 19.1 ist ein Pulsweite-Spannungsregler 19.2 integriert, welcher über ein LC-Glied eine Betriebsspannung von ca. 50 bis 150 Volt für die Primärspule eines Hochspannungstransformators 19.3 zur Verfügung stellt, dessen Sekundär-Spule an einen Luftionisator 19.4 gelegt ist. Die Primärspule liegt im Kollektor-Emitter-Kreis eines Leistungstransistor 19.5 zur impulsförmigen Hochspannungserzeugung. Eine Ansteuerschaltung 19.6, welche die Basisstrecke des Schalttransistors ansteuert, dient zur Schaltung des Transistors 19.5. Die Betriebsspannung durchfließt die Primärspule des Hochspannungstransformators 19.3, wenn der Leistungstransistor 19.5 durchgeschaltet ist. Die Ansteuerung des Leistungstransistors 19.5 erfolgt durch die Ansteuerschaltung 19.6 mit einer bestimmten Frequenz und einem bestimmten Puls-Pause-Verhältnis. In bevorzugter Ausführung wird eine Frequenz von 15 bis 20 khz gewählt und ein Einschaltverhältnis von 15 %.

Eine Steuer- und Regeleinheit 19.7 erzeugt eine sägezahnförmige Spannung, welche den Pulsweite-Spannungsregler 19.2 beeinflusst und dessen Ausgangsspannung sukzessive erhöht. Ein Ozonsensor 19.8 ist mit der Steuer- und Regeleinheit 19.7 verbunden und wirkt so auf diese ein, dass bei Auftreten von Ozon die Regelspannung und damit die Hochspannung sofort auf ein Maß zurückgesetzt wird, welches sicher keine Ozonproduktion mehr zulässt.

### Gewerbliche Anwendbarkeit:

Die Erfindung ist insbesondere gewerblich für die Luftreinhaltung, insbesondere von Atemluft, einsetzbar. Die Nützlichkeit der Erfindung besteht insbesondere darin, daß der Luftionisator immer ein Optimum an Sauerstoffionen zu erzeugen imstande ist, ohne jedoch dabei Ozon zu produzieren.

## Patentansprüche

1. Vorrichtung zur Erzeugung aktiver Sauerstoffionen in der Luft für die Luftverbesserung, insbesondere von Atemluft, bestehend aus wenigstens einem Luftionisator und einem eine zur Luftionisierung ausreichende elektrische Hochspannung erzeugenden elektrischen Transformator, mit einem den Gehalt der Luft an oxidierbaren Gasen feststellenden Luftgütesensor und einer elektrischen Steuereinrichtung, die aufgrund des ermittelten Gehaltes derartiger oxidierbare Gase die dem Luftionisator zugeführte elektrische Energie derart verändert, daß bei niedrigen Konzentrationen oxidierbarer Gase eine nur geringe Ionisationsleistung erfolgt, welche sensorgesteuert und automatisch mit zunehmenden Konzentrationen an oxidierbaren Gasen auf einen Maximumwert steigerbar ist.

2. Vorrichtung nach Anspruch 1, **dadurch gekennzeichnet,**
**daß** ein nach dem Luftionisator angeordneter Ionenzähler die Anzahl der in der Luft befindlichen Ionen detektiert und über eine elektronische Schaltung so auf die Vorrichtung bzw. den Luftionisator einwirkt, dass sensorgesteuert und automatisch, vorzugsweise stetig, bei geringer Ionenzahl die Ionisationsleistung des Luftionisators erhöht und bei erhöhter Ionenzahl verringert wird.

3. Vorrichtung nach Anspruch 1 oder 2, **dadurch gekennzeichnet,**
**dass** zur Änderung der Ansteuerleistung des Luftionisators der Transformator verschiedene Wicklungsanzapfungen aufweist und über dieselben so ansteuerbar ist, dass sich wirkungsrichtig und sensorgesteuert eine höhere oder niedrige Betriebsspannung des Luftionisators ergibt.

4. Ionisationsapparat nach Anspruch 1 oder 2, **dadurch gekennzeichnet,**
**dass** zur Änderung der Ansteuerleistung des Luftionisators demselben eine Kette von kapazitiven oder ohmschen Widerständen vorgeschaltet ist, die durch geeignete Schaltglieder wirkungsrichtig so überbrückt werden, dass sich entsprechend der Überbrückung eine angepaßte Ionisationsleistung des Luftionisators ergibt.

5. Ionisationsapparat nach Anspruch 1 oder 2, **dadurch gekennzeichnet,**
**dass** die wirkungsrichtige und situationsangepaßte Änderung der Ionisationsleistung erreicht wird, indem eine Vielzahl von Luftionisatoren vorhanden sind und betrieben werden und dabei die aktive Fläche der betriebenen Luftionisatoren durch geeignete elektrische Schaltglieder den durch den bzw. die Luftgütesensoren ermittelten Erfordernissen angepaßt wird.

6. Vorrichtung nach Anspruch 4 oder 5, **dadurch gekennzeichnet,**
**dass** die Anhebung der Ionisationsleistung sensorgesteuert jeweils dann erfolgt, wenn die Änderung der vom Luftgütesensor detektierten Gaskonzentration einen bestimmten Quotienten über die Zeit aufweist.

7. Vorrichtung nach Anspruch 1, **dadurch gekennzeichnet,**
**dass** die Anhebung der Ionisationsleistung des Luftionisators sensorgesteuert jeweils dann erfolgt, wenn der gasabhängige Wert des Luftgütesensors oder der Quotient aus der Wertänderung des Luftgütesensors über die Zeit einen bestimmten Wert überschritten hat.

8. Vorrichtung nach Anspruch 1, **dadurch gekennzeichnet,**
**daß** der Luftionisators einem Luftbefeuchter vorgeschaltet ist.

9. Vorrichtung nach Anspruch 1, **dadurch gekennzeichnet,**
**daß** dem Luftionisator ein Ozonsensor nachgeschaltet ist, welcher mit der elektrischen Steuerschaltung verbunden ist und welcher bei Feststellung eines bestimmten Ozongehaltes in der Luft auf die elektrische Steuerschaltung einwirkt, die die dem Luftionisator zugeführte elektrische Energie verringert.

10. Vorrichtung nach Anspruch 9, **dadurch gekennzeichnet,**
**daß** zur Steuerung der dem Luftionisator zugeführten elektrische Energie beim Auftreten von Ozon eine Ansteuerung mit einer sägezahnförmigen Spannung erfolgt, die bei Erreichen einer die Ozonproduktion zulassenden Spannung auf eine solche Spannung zurückgeführt wird, bei welcher einerseits sicher ionisiert, andererseits aber noch nicht ozonisiert wird.

11. Vorrichtung nach Anspruch 10, **dadurch gekennzeichnet,**
**daß** die sägezahnförmigen Spannung innerhalb eines Spannungsbandes zwischen den beiden Spannungspegeln rampenförmig oder sägezahnförmig hin und her pendelt.

12. Vorrichtung nach Anspruch 1, **dadurch gekennzeichnet,**
**daß** der Luftionisator aus zwei oder mehreren elektrisch leitenden, flächigen oder plattenförmigen Strukturkörpern als Elektroden besteht, die sich planparallel gegenüberstehen und die durch ein Dielektrikum elektrisch voneinander getrennt sind und einen flächigen Kondensator bilden, wobei an die Elektroden eine zur Ionisation von Luft ausreichend hohe Wechselspannung gelegt ist.

13. Vorrichtung nach Anspruch 12, **dadurch gekennzeichnet,**
**dass** der Luftionisator aus einer flächigen äußeren und einer flächigen inneren Elektrode besteht, die hermetisch von einem die äußere Elektrode tragendes Dielektrikum eingeschlossen ist, wobei die äußere und innere Elektrode je durch einen elektrischen Anschluß kontaktiert sind, und an die innere und die äußere Elektrode eine zur Ionisation von Luft ausreichend hohe elektrische Wechselspannung angeschlossen ist.

14. Vorrichtung nach Anspruch 12 oder 13, **dadurch gekennzeichnet,**
**dass** eine Mehrzahl von flachen Luftionisatoren (Flachionisator) zu einem Stapel oder zu mehreren elektrisch voneinander isolierten Stapeln geschichtet sind, die von der zu behandelnden Luft durchströmt werden und dass an die einzelnen Elektroden eine zur Ionisation von Luft ausreichend hohe elektrische Wechselspannung angelegt wird.

15. Vorrichtung nach Anspruch 12 oder 13, **dadurch gekennzeichnet,**
**daß** die inneren Elektroden das gleiche elektrische Potential wie die benachbarten Flachionisatoren haben oder die inneren Elektroden ein den benachbarten Flachionisatoren ungleiches elektrisches Potential aufweisen.

16. Vorrichtung nach Anspruch 12, **dadurch gekennzeichnet,**
**daß** der Luftionisator luftdurchlässig ist, wobei die beiden flächigen Elektroden Durchbrechungen aufweisen und gitterartig oder lochartig strukturiert sind und die zu ionisierende Luft den freien Querschnitt der Elektroden durchströmt.

17. Vorrichtung nach Anspruch 16, **dadurch gekennzeichnet,**
**daß** eine der beiden Elektroden aus einem elektrisch leitfähigen Filtermaterial besteht, welches von der anderen Elektrode gitterartig, aber elektrisch isoliert, umgeben ist und die zu behandelnde Luft beide Elektroden durchströmt.

18. Vorrichtung nach Anspruch 12, **dadurch gekennzeichnet,**
**daß** mindestens eine der Elektroden eine Vielzahl von gegen die Gegenelektrode gerichteten Nadeln oder Spitzen oder Zacken aufweist.

19. Vorrichtung nach Anspruch 12, **dadurch gekennzeichnet,**
**dass** ein Stapel aus Ionisationsplatten in einem Belüftungskanal angeordnet ist, und den gesamten Querschnitt des Belüftungskanals ausfüllt und dergestalt einen Luftionisator bildet und die Ionisationsplatten entweder mit der schmalseitigen Stirnfläche oder mit ihrer luftdurchströmbaren Querschnittsfläche gegen die Luftströmung hin ausgerichtet sind.

20. Verwendung einer Vorrichtung nach Anspruch 1 oder 12 oder 19,
**dadurch gekennzeichnet,**
**daß** dieselbe in einem Außenluft fördernden Luftkanal eines Kraftfahrzeuges eingebaut ist.

## Claims

1. A device to produce active oxygen ions in the air for purposes of improving air quality, particularly breathing air, consisting of at least one air ionizer and an electric transformer capable of generating sufficient electric high-voltage for the ionization of the air, having an air air-quality sensor that ascertains the level of oxidizable gases in the air and having an electric control unit which, on the basis of the ascertained level of such oxidizable gases, then changes the electric power being supplied to the air ionizer in such a way that, if the concentration of oxidizable gases is low, only a small ionization output is generated which can be increased to a maximum value in a sensor-controlled and automatic manner as the concentration of oxidizable gases rises.

2. The device according to Claim 1, **characterized in that**
an ion counter located downstream from the air ionizer detects the number of ions present in the air and, by means of an electronic circuit, then acts upon the device or air ionizer in such a way that, in a sensor-controlled and automatic manner, the ionization output is increased if the ion count is low and decreased if the ion count is high.

3. The device according to Claim 1 or 2, **characterized in that**,
in order to change the controlling output of the air ionizer, the transformer has several coil extraction points by means of which it can be controlled in such a way that the air ionizer has a higher or lower operating voltage in a manner that is effective and sensor-controlled.

4. The ionization apparatus according to Claim 1 or 2, **characterized in that**,
in order to change the controlling output of the air ionizer, there is a chain of capacitive or ohmic resistors located upstream from the air ionizer, and said resistors are effectively bridged by means of suitable switching elements in such a way that, as a function of the bridging, the ionization output of the air ionizer is adjusted.

5. The ionization apparatus according to Claim 1 or 2, **characterized in that**
the effective and situation-adjusted change of the ionization output is achieved **in that** a plurality of air ionizers are present and are operated, whereby the active surface area of the operated air ionizers is adapted by means of appropriate electric switching elements to the requirements ascertained by the air-quality sensor or sensors.

6. The device according to Claim 4 or 5, **characterized in that**
the ionization output is increased in a sensor-controlled manner whenever the change in the gas concentration detected by the air-quality sensor has a certain quotient over time.

7. The device according to Claim 1, **characterized in that**
the ionization output of the air ionizer is increased in a sensor-controlled manner whenever the gas-dependent value indicated by the air-quality sensor or the quotient from the value change of the air-quality sensor over time exceeds a certain value.

8. The device according to Claim 1, **characterized in that**
a humidifier is installed upstream from the air ionizer.

9. The device according to Claim 1, **characterized in that**, downstream from the air ionizer, there is an ozone sensor that is connected to the electric control unit and that, upon ascertaining a certain ozone level in the air, acts upon the electric control circuit, which then reduces the electric power that is being supplied to the air ionizer.

10. The device according to Claim 9, **characterized in that**,
in order to control the electric power that is being supplied to the air ionizer when the ozone occurs, a control operation is carried out with a sawtooth voltage which, when a voltage level that allows ozone production is reached, is reduced to such a level at which, on the one hand, ionization reliably takes place while, on the other hand, ozonization does not yet occur.

11. The device according to Claim 10, **characterized in that**
the sawtooth voltage oscillates ramp-like or sawtooth-like within a voltage band between the two voltage levels.

12. The device according to Claim 1, **characterized in that**
the air ionizer consists of two or more electrically conductive, flat or laminar structural elements serving as electrodes, which lie plane-parallel opposite from each other and which are electrically separated from each other by a dielectric material and which form a flat condenser, whereby an alternating current that is sufficiently strong for ionization of the air is applied to the electrodes.

13. The device according to Claim 12, **characterized in that**
the air ionizer consists of a flat external electrode and a flat internal electrode that is hermetically enclosed by a dielectric material that supports the external electrode, whereby the external and the internal electrodes are each contacted by means of an electric connection and whereby an alternating current that is sufficiently strong to ionize the air is applied to the internal and external electrodes.

14. The device according to Claim 12 or 13, **characterized in that**
a plurality of flat air ionizers (flat ionizers) are layered to form a stack or to form several stacks that are electrically insulated from each other, through which the air to be treated passes, and **in that** an alternating current that is sufficiently strong to ionize the air is applied to the individual electrodes.

15. The device according to Claim 12 or 13, **characterized in that**
the internal electrodes have the same electric potential as the adjacent flat ionizers or else the internal electrodes display an electric potential that differs from that of the adjacent flat ionizers.

16. The device according to Claim 12, **characterized in that**
the air ionizer is air-permeable, whereby the two flat electrodes have cutouts and are structured like a lattice or with perforations so that the air to be ionized can pass through the free cross section of the electrodes.

17. The device according to Claim 16, **characterized in that**
one of the two flat electrodes is made of an electrically conductive filter material that is surrounded by the other electrode in a lattice-like and yet electrically insulated manner so that the air to be treated can pass through both electrodes.

18. The device according to Claim 12, **characterized in that**
at least one of the electrodes has a plurality of needles or points or spikes aligned opposite to the counter electrode.

19. The device according to Claim 12, **characterized in that**
a stack consisting of ionization plates is arranged in a ventilation duct and it occupies the entire cross section of the ventilation duct, thus forming an air ionizer, and either the narrow face of the ionization plates or their cross section surfaces through which the air flows are aligned opposite to the direction of flow.

20. The use of the device according to Claim 1 or 12 or 19,
**characterized in that** said device is installed in a vehicle air duct that serves to convey outside air.

## Revendications

1. Dispositif de production d'ions d'oxygène actifs dans l'air pour la purification de l'air, notamment de l'air que l'on respire, consistant en au moins un ionisateur d'air et un transformateur électrique produisant une haute tension électrique suffisante pour l'ionisation de l'air, avec un détecteur de la qualité de l'air qui détermine la teneur en gaz oxydables contenus dans l'air et un appareil de commande électrique qui, compte tenu de la teneur en gaz oxydables de cette sorte déterminée, modifie l'énergie électrique amenée à l'ionisateur d'air de sorte qu'en présence de basses concentrations de ces gaz oxydables, il s'ensuit seulement une faible puissance d'ionisation qui, par commande du détecteur, peut automatiquement s'élever jusqu'à atteindre une valeur maxima au fur et à mesure que les concentrations en gaz oxydables augmentent.

2. Dispositif selon la revendication 1, **caractérisé en ce que**
un compteur d'ions installé en aval de l'ionisateur d'air détecte le nombre d'ions se trouvant dans l'air et agit via un circuit de commande électronique de telle sorte sur le dispositif, en l'occurrence sur l'ionisateur d'air, de façon à ce que, par commande du détecteur et automatiquement, de préférence de façon continue, la puissance d'ionisation de l'ionisateur d'air augmente lorsque les ions sont en bas nombre et diminue lorsque les ions sont en nombre élevé.

3. Dispositif selon l'une des revendications 1 ou 2, **caractérisé en ce que**
afin de modifier la puissance d'amorçage de l'ionisateur d'air, le transformateur présente diverses prises d'enroulement au moyen desquelles il est commandé de façon à ce que, en produisant l'effet voulu et par commande du détecteur, il résulte une tension de travail de l'ionisateur d'air qui est plus élevée ou plus basse.

4. Appareil d'ionisation selon l'une des revendications 1 ou 2,
**caractérisé en ce que** pour modifier la puissance d'amorçage de l'ionisateur d'air, une série de résistances capacitives ou ohmiques sont connectées en amont de ce dernier, ces résistances étant pontées par des organes de commande électrique appropriés qui produisent l'effet voulu, de façon à ce qu'il en résulte une puissance d'ionisation de l'ionisateur d'air adaptée, conformément au pontage.

5. Appareil d'ionisation selon l'une des revendications 1 ou 2,
**caractérisé en ce que** l'on obtient la modification de la puissance d'ionisation qui produit l'effet voulu et est adaptée à la situation, dans la mesure où l'on est en présence d'un grand nombre d'ionisateurs d'air qui sont en service, la surface active des ionisateurs d'air qui sont en marche étant adaptée par le truchement d'organes de commande électriques appropriés aux conditions requises qui ont été déterminées par les détecteurs de la qualité de l'air.

6. Dispositif selon l'une des revendications 4 ou 5, **caractérisé en ce que**
l'augmentation de la puissance d'ionisation commandée par le détecteur se produit à chaque fois que la modification de la concentration de gaz détectée par le détecteur de la qualité de l'air fait état d'un certain quotient dans le temps.

7. Dispositif selon la revendication 1, **caractérisé en ce que**
l'augmentation de la puissance d'ionisation de l'ionisateur d'air commandée par le détecteur se produit à chaque fois que la valeur qui est fonction du gaz dont fait état le détecteur de la qualité de l'air, ou que le quotient résultant de la modification de la valeur du détecteur de la qualité de l'air a dépassé une certaine valeur dans le temps.

8. Dispositif selon la revendication 1, **caractérisé en ce que**
l'ionisateur d'air est installé en amont d'un humidificateur d'air.

9. Dispositif selon la revendication 1, **caractérisé en ce que**
un détecteur d'ozone relié à un circuit de commande électrique est connecté en aval de l'ionisateur d'air et qui, lorsqu'il détecte une certaine teneur en ozone dans l'air, agit sur de circuit de commande électrique qui diminue l'énergie électrique amenée à l'ionisateur d'air.

10. Dispositif selon la revendication 9, **caractérisé en ce que**
pour la commande de l'énergie électrique amenée à l'ionisateur d'air en présence d'ozone, il se produit un amorçage avec une tension en forme de dents de scie qui lors de l'obtention d'une tension qui permet la production d'ozone, est ramenée à une tension telle qui d'une part, ionise en tout état de cause, et d'autre part, ne produit pas encore d'ozone.

11. Dispositif selon la revendication 10, **caractérisé en ce que**
la tension en forme de dents de scie oscille dans une bande de tension entre les deux niveaux de tension, en dents de scie ou en rampe.

12. Dispositif selon la revendication 1, **caractérisé en ce que**
l'ionisateur d'air consiste en deux ou plusieurs corps structuraux électroconducteurs, plans ou en forme de plaques en tant qu'électrodes, se faisant face en parallélisme parfait et coupés électriquement l'un de l'autre par un diélectrique et formant un condensateur plan, une tension alternative suffisamment élevée pour l'ionisation de l'air étant appliquée aux électrodes.

13. Dispositif selon la revendication 12, **caractérisé en ce que**
l'ionisateur d'air consiste en une électrode plane extérieure et une électrode plane intérieure, cette dernière étant hermétiquement entourée par un diélectrique portant l'électrode extérieure, un contact étant établi avec chacune des électrodes extérieure et intérieure par un raccordement électrique et une tension alternative suffisamment élevée pour l'ionisation de l'air étant appliquée à l'électrode intérieure et extérieure.

14. Dispositif selon l'une des revendications 12 ou 13, **caractérisé en ce que**
une pluralité d'ionisateurs d'air plats (ionisateurs plats) sont disposés en couches pour former une pile ou plusieurs piles électriquement isolées et à travers lesquelles passe l'air qui doit être traité, et qu'une tension alternative suffisamment élevée pour l'ionisation de l'air est appliquée à chacune des électrodes.

15. Dispositif selon l'une des revendications 12 ou 13, **caractérisé en ce que**
les électrodes intérieures ont le même potentiel électrique que les ionisateurs plats voisins ou que les électrodes intérieures font état d'un potentiel électrique inégal à celui des ionisateurs plats voisins.

16. Dispositif selon la revendication 12, **caractérisé en ce que**
l'ionisateur d'air laisse passer l'air, les deux électrodes planes présentant des percées et ont une structure en grille ou à perforations et que l'air à ioniser traverse la section libre des électrodes.

17. Dispositif selon la revendication 16, **caractérisé en ce que**
l'une des deux électrodes consiste en un matériau filtre électroconducteur qui est entouré par l'autre électrode comme une sorte de grillage, mais étant électriquement isolé et que l'air qui doit être traité traverse les deux électrodes.

18. Dispositif selon la revendication 12, **caractérisé en ce que**
au moins l'une des électrodes présente un grand nombre d'aiguilles, de picots, ou de dentelures dirigés vers la contre-électrode.

19. Dispositif selon la revendication 12, **caractérisé en ce que**
une pile constituée de plaques d'ionisation est disposée dans un canal d'aération et remplit toute la section du canal d'aération constituant de la sorte un ionisateur d'air et que les plaques d'ionisation sont orientées de façon à présenter ou bien le côté tranche ou bien le côté plat au courant d'air.

20. Utilisation d'un dispositif selon l'une des revendiactions 1, 12 ou 19, **caractérisé en ce que** ledit dispositif est monté dans un canal d'air d'un véhicule qui conduit de l'air de l'extérieur.
